# EUROPEAN PATENT APPLICATION

(11) **EP 1 852 116 A1**
(43) Date of publication of application: **07.11.2007**
(21) Application number: 07110366.7
(22) Date of filing: 29.07.2002
(51) Int. Cl.: A61K 31/40, A61K 31/44, A61P 9/00, A61P 9/08, A61P 9/10, A61P 9/12, A61P 9/14

(54) **Pharmaceutical compositions of amlodipine and atorvastatin**

(30) Priority: 31.07.2001 US 309133 P
(62) Divisional of application: 02751483.5
(71) Applicant: Warner-Lambert Company LLC, New York, NY 10017 (US)
(72) Inventor: Alani, Laman, Ann Arbor, MI 48105 (US); Khan, Sadath, Ulla, Ann Arbor, MI 48105 (US); Macneil, Thomas, Michael, Ann Arbor, MI 48105 (US); Muhammad, Nouman, Abdul-Hussain, Ann Arbor, MI 48105 (US)
(74) Representative: Rudge, Andrew John

(57) **Abstract**

The present invention relates to a pharmaceutical composition comprising two components: one component comprising a granulation of atorvastatin or pharmaceutically acceptable salts thereof and a carrier including an alkalizing agent that forms a pH greater than 5; and a second component comprising amlodipine or pharmaceutically acceptable salts thereof and a carrier excluding an alkalizing agent that forms a pH greater than 5; wherein the two components are combined to form a final composition for a solid dosage form.

## Description

### FIELD OF THE INVENTION

This invention relates to pharmaceutical compositions comprising amlodipine and pharmaceutically acceptable salts thereof, and atorvastatin and pharmaceutically acceptable salts thereof, and a process for the preparation of the same, kits containing such compositions, as well as methods of using such compositions to treat subjects suffering from angina pectoris, atherosclerosis, combined hypertension and hyperlipidemia and/or hypercholesterolemia and to treat subjects presenting with symptoms of cardiac risk, including human subjects.

### BACKGROUND OF THE INVENTION

The conversion of 3-hydroxy-3-methylglutaryl-coenzyme A (HMG-CoA) to mevalonate is an early and rate-limiting step in the cholesterol biosynthetic pathway. This step is catalyzed by the enzyme HMG-CoA reductase. Statins inhibit HMG-CoA reductase from catalyzing this conversion. As such, statins are collectively potent lipid lowering agents.

Atorvastatin calcium, disclosed in United States Patent No. 5,273,995 which is incorporated herein by reference, is currently sold as Lipitor® having the chemical name [R-(R*,R*)]-2-(4-fluorophenyl)-β,δ-dihydroxy-5-(1-methylethyl)-3-phenyl-4-[(phenylamino)carbonyl]-1H-pyrrole-1-heptanoic acid calcium salt (2:1) trihydrate and the formula

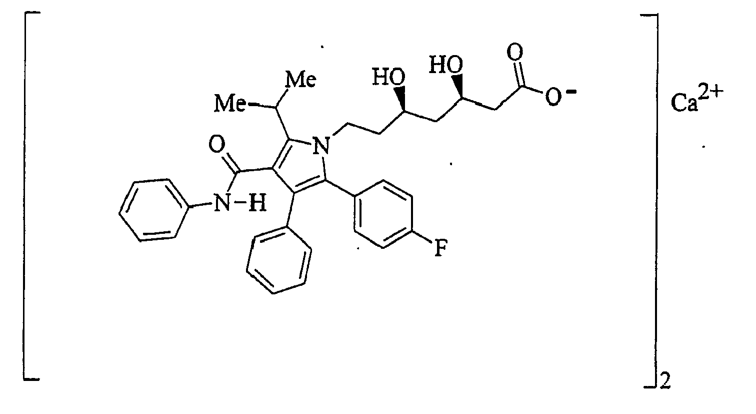

Atorvastatin and pharmaceutically acceptable salts thereof are selective, competitive inhibitors of HMG-CoA reductase. As such, atorvastatin calcium is a potent lipid lowering compound and is thus useful as a hypolipidemic and/or hypocholesterolemic agent.

United States Patent Number 4,681,893, which is incorporated herein by reference, discloses certain *trans-*6-[2-(3- or 4-carboxamido-substituted-pyrrol-1-yl)alkyl]-4-hydroxy-pyran-2-ones including *trans* (±)-5-(4-fluorophenyl)-2-(1-methylethyl)-N, 4-diphenyl-1-[(2-tetrahydro-4-hydroxy-6-oxo-2H-pyran-2-yl)ethyl]-1 H-pyrrole-3-carboxamide.

United States Patent Number 5.273,995, which is herein incorporated by reference, discloses the enantiomer having the R form of the ring-opened acid of *trans-*5*-*(4-fluorophenyl)-2-(1-methylethyl)-N, 4-diphenyl-1-[(2-tetrahydro-4-hydroxy-6-oxo-2H-pyran-2-yl)ethyl]- 1H-pyrrole-3-carboxamide, ie, [R-(R*,R*)]-2-(4-fluorophenyl)-β,δ-dihydroxy-5-(1-methylethyl)-3-phenyl-4-[(phenylamino)-carbonyl]-1H-pyrrole-1-heptanoic acid which is atorvastatin.

United States Patent Numbers 5,003,080, 5,097,045; 5,103,024; 5,124,482; 5,149,837; 5,155.251; 5,216,174; 5.245,047; 5,248,793; 5,280,126; 5,397,792; 5.342,952: 5.298,627; 5.446,054; 5.470.981; 5,489,690; 5,489,691; 5,510,488; 5.998.633; and 6.087,511, which are herein incorporated by reference, disclose various processes and key intermediates for preparing atorvastatin.

Crystalline forms of atorvastatin calcium are disclosed in United States Patent Numbers 5,969,156 and 6.121.461 which are herein incorporated by reference.

Stable oral formulations of atorvastatin calcium are disclosed in United States Patent Numbers 5.686,104 and 6,126,971.

Amlodipine and related dihydropyridine compounds are disclosed in United States Patent Number 4,572,909, which is incorporated herein by reference, as potent anti-ischemic and antihypertensive agents. United States Patent Number 4,879,303, which is incorporated herein by reference, discloses amlodipine benzenesulfonate salt (also termed amlodipine besylate). Amlodipine and amlodipine besylate are potent and long-lasting calcium channel blockers. As such, amlodipine, amlodipine besylate and other pharmaceutically acceptable acid addition salts of amlodipine have utility as antihypertensive agents and as anti-ischemic agents. Amlodipine and its pharmaceutically acceptable acid addition salts are also disclosed in United States Patent Number 5,155,120 as having utility in the treatment of congestive heart failure. Amlodipine besylate is currently sold as Norvasc®. Amlodipine has the formula

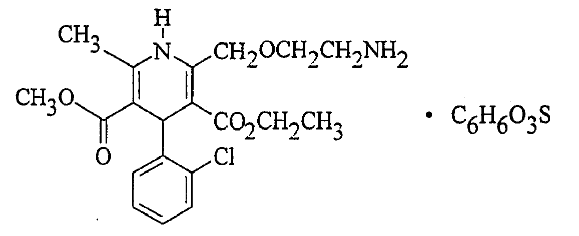

Atherosclerosis is a condition characterized by irregularly distributed lipid deposits in the intima of arteries, including coronary, carotid and peripheral arteries. Atherosclerotic coronary heart disease (hereinafter termed "CHD") accounts for 53% of all deaths attributable to a cardiovascular event. CHD accounts for nearly one-half (about $50-$60 billion) of the total United States cardiovascular healthcare expenditures and about 6% of the overall national medical bill each year. Despite attempts to modify secondary risk factors such as, *inter alia,* smoking, obesity and lack of exercise, and treatment of dyslipidemia with dietary modification and drug therapy, CHD remains the most common cause of death in the United States.

High levels of blood cholesterol and blood lipids are conditions involved in the onset of atherosclerosis. It is well-known that inhibitors of 3-hydroxy-3-methylglutaryl-coenzyme A reductase (HMG-CoA reductase) are effective in lowering the level of blood plasma cholesterol, especially low density lipoprotein cholesterol (LDL-C), in man (Brown and Goldstein, New England Journal of Medicine, 1981;305(9):515-517). It has now been established that lowering LDL-C levels affords protection from coronary heart disease (see e.g., The Scandinavian Simvastatin Survival Study Group. Randomised trial of cholesterol lowering in 4444 patients with coronary heart disease: the Scandinavian Simvastatin Survival Study (4S), Lancet, 1994;344:1383-1389; and Shepherd J. et al., Prevention of coronary heart disease with pravastatin in men with hypercholesterolemia, New England Journal of Medicine, 1995;333:1301-1307).

Angina pectoris is a severe constricting pain in the chest, often radiating from the precordium to the left shoulder and down the left arm. Often angina pectoris is due to ischemia of the heart and is usually caused by coronary disease.

Currently, the treatment of symptomatic angina pectoris varies significantly from country to country. In the United States, patients who present with symptomatic, stable angina pectoris are frequently treated with surgical procedures or Percutaneous Transluminal Coronary Angioplasty (PTCA). Patients who undergo PTCA or other surgical procedures designed to treat angina pectoris frequently experience complications such as restenosis. This restenosis may be manifested either as a short-term proliferative response to angioplasty-induced trauma or as long-term progression of the atherosclerotic process in both graft vessels and angioplastied segments.

The symptomatic management of angina pectoris involves the use of a number of drugs, frequently as a combination of two or more of the following classes: beta blockers, nitrates and calcium channel blockers. Most, if not all, of these patients require therapy with a lipid lowering agent as well. The National Cholesterol Education Program (NCEP) recognizes patients with existing coronary artery disease as a special class requiring aggressive management of raised LDL-C.

Amlodipine helps to prevent myocardial ischemia in patients with exertional angina pectoris by reducing Total Peripheral Resistance, or afterload, which reduces the rate pressure product and thus myocardial oxygen demand at any particular level of exercise. In patients with vasospastic angina pectoris, amlodipine has been demonstrated to block constriction and thus restore myocardial oxygen supply. Further, amlodipine has been shown to increase myocardial oxygen supply by dilating the coronary arteries.

Hypertension frequently coexists with hyperlipidemia and both are considered to be major risk factors for developing cardiac disease ultimately resulting in adverse cardiac events. This clustering of risk factors is potentially due to a common mechanism. Further, patient compliance with the management of hypertension is generally better than patient compliance with hyperlipidemia. It would therefore be advantageous for patients to have a single therapy which treats both of these conditions.

Coronary heart disease is a multifactorial disease in which the incidence and severity are affected by the lipid profile, the presence of diabetes, and the sex of the subject. Incidence is also affected by smoking and left ventricular hypertrophy which is secondary to hypertension. To meaningfully reduce the risk of coronary heart disease, it is important to manage the entire risk spectrum. For example, hypertension intervention trials have failed to demonstrate full normalization in cardiovascular mortality due to coronary heart disease. Treatment with cholesterol synthesis inhibitors in patients with and without coronary artery disease reduces the risk of cardiovascular morbidity and mortality.

The Framingham Heart Study, an ongoing prospective study of adult men and women, has shown that certain risk factors can be used to predict the development of coronary heart disease (see Wilson et al., Am. J. Cardiol., 1987;59(14):91G-94G). These factors include age, gender, total cholesterol level, high density lipoprotein (HDL) level, systolic blood pressure, cigarette smoking, glucose intolerance, and cardiac enlargement (left ventricular hypertrophy on electrocardiogram, echocardiogram or enlarged heart on chest x-ray). Calculators and computers can easily be programmed using a multivariate logistic function that allows calculation of the conditional probability of cardiovascular events. These determinations, based on experience with 5,209 men and women participating in the Framingham study, estimate coronary artery disease risk over variable periods of follow-up. Modeled incidence rates range from less than 1 % to greater than 80% over an arbitrarily selected 6-year interval. However, these rates are typically less than 10% and rarely exceed 45% in men and 25% in women.

Kramsch et al., Journal of Human Hypertension, 1995;(Suppl. 1):53-59, disclose the use of calcium channel blockers, including amlodipine, to treat atherosclerosis. That reference further suggests that atherosclerosis can be treated with a combination of amlodipine and a lipid lowering agent. Human trials have shown that calcium channel blockers have beneficial effects in the treatment of early atherosclerotic lesions (see e.g., Lichtlen P.R. et al., Retardation of angiographic progression of coronary artery disease by nifedipine, Lancet, 1990;335:1109-1113; and Waters D. et al., A controlled clinical trial to assess the effect of a calcium channel blocker on the progression of coronary atherosclerosis, Circulation, 1990;82:1940-1953). United States Patent Number 4,681,893 discloses that certain statins, including atorvastatin, are hypolipidemic agents and as such are useful in treating atherosclerosis. Jukema et al., Circulation, 1995;(Suppl. 1):1-197, disclose that there is evidence that calcium channel blockers act synergistically in combination with lipid lowering agents (e.g., HMG-CoA reductase inhibitors), specifically pravastatin. Orekhov et al., Cardiovascular Drugs and Therapy, 1997;11:350, disclose the use of amlodipine in combination with lovastatin for the treatment of atherosclerosis.

International Published Patent Application WO 99/11259 discloses therapeutic combinations comprising amlodipine and atorvastatin. Thus, it is desirable to be able to administer these two pharmaceutical agents to a patient in need of dual therapy. Furthermore, it is even more desirable to be able to administer both of these agents in a single dosage form.

Therefore, it is an object of the present invention to provide a stable dosage form having good bioavailability. It is a further object of the present invention to provide a stable composition with low levels of impurities and/or degradants that may occur during preparation and/or subsequent storage of the composition. We have surprisingly and unexpectedly found that amlodipine and atorvastatin can be formulated in a single dosage form that is stable, has bioavailability equivalent to administering each therapeutic agent in a separate dosage form, and contains very low levels of impurities and/or degradants despite the known incompatibilities between amlodipine and atorvastatin.

### SUMMARY OF THE INVENTION

Accordingly, the first aspect of the present invention is a pharmaceutical composition comprising two components:
(a) one component comprising a granulation of atorvastatin or pharmaceutically acceptable salts thereof and a carrier including an alkalizing agent that forms a pH greater than 5; and
(b) a second component comprising amlodipine or pharmaceutically acceptable salts thereof and a carrier excluding an alkalizing agent-that forms a pH greater than 5, wherein the two components are combined to form a final composition for a solid dosage form.

A second aspect of the present invention is a method for preparing a pharmaceutical composition comprising:
[A] An atorvastatin granulation comprising:
   Step (1)- dissolving a surface active agent in water and adding and hydrating a binder;
   Step (2) - mixing atorvastatin calcium, an alkalizing agent that forms a pH greater than 5, a filler/diluent, a filler/diluent/disintegrating agent, and a disintegrating agent in a granulating apparatus;
   Step (3) - granulating the powder mix from Step (2) with the solution from Step (1) in the granulating apparatus; and
   Step (4) - drying the granulation in a drying apparatus;
[B] A final formulation comprising:
   Step (1) - adding amlodipine besylate, a filler/diluent, a disintegrating agent, and a glidant to the atorvastatin formulation;
   Step (2) - passing the powder mixture through a mill; and
   Step (3) - blending the milled powder mixture and a lubricating agent in a blender to afford a uniformly blended pharmaceutical composition for a solid dosage form.

A third aspect of the present invention is a pharmaceutical composition having low levels of degradation products and/or impurities.

A fourth aspect of the present invention is a method of using the pharmaceutical compositions to treat subjects suffering from angina pectoris, atherosclerosis, combined hypertension and hyperlipidemia and/or hypercholesterolemia, and to treat subjects presenting with symptoms of cardiac risk, including human subjects.

A fifth aspect of the present invention is a therapeutic package or kit suitable for commercial sale, comprising a container and a pharmaceutical composition having low levels of degradation products and/or impurities.

### DESCRIPTION OF THE DRAWINGS

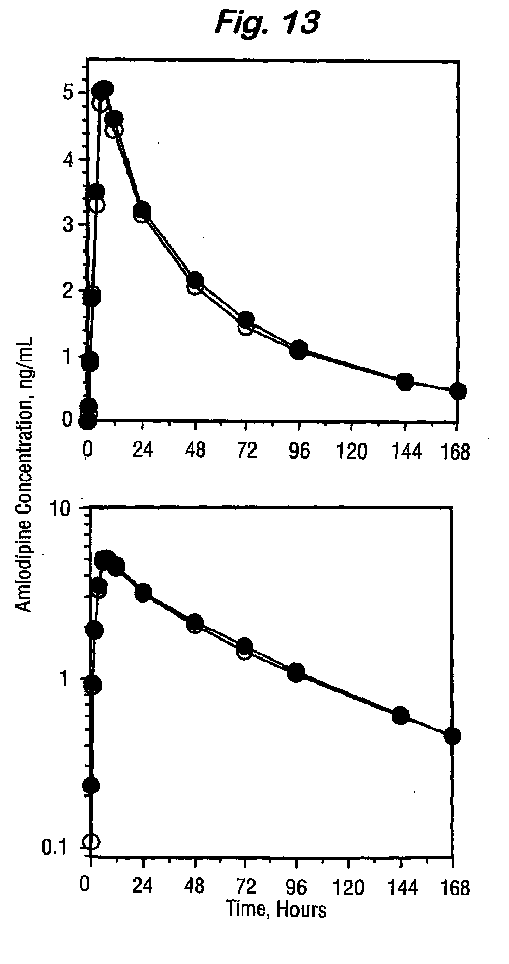

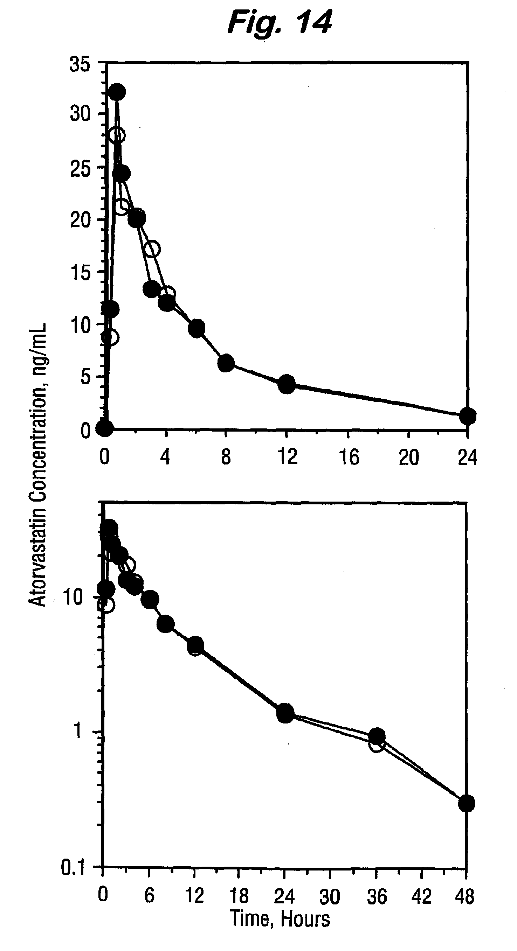

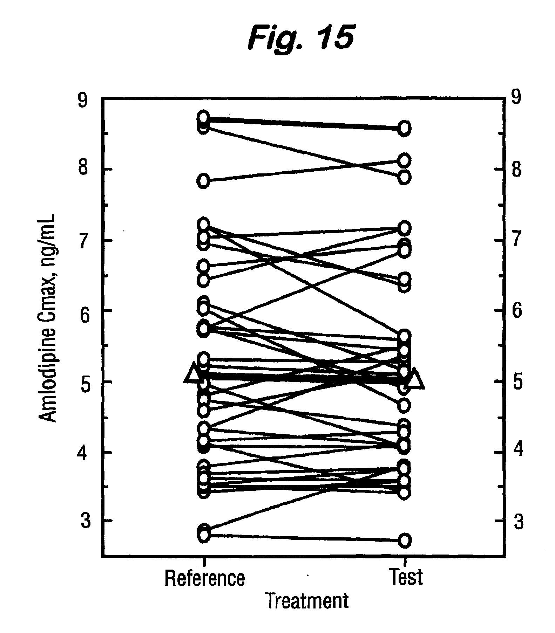

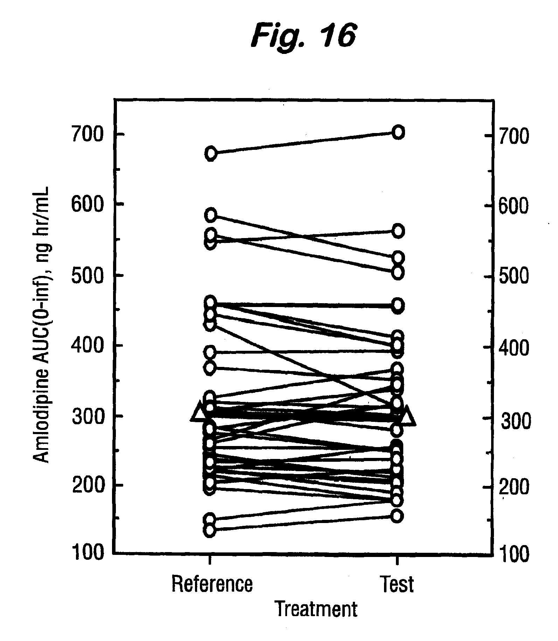

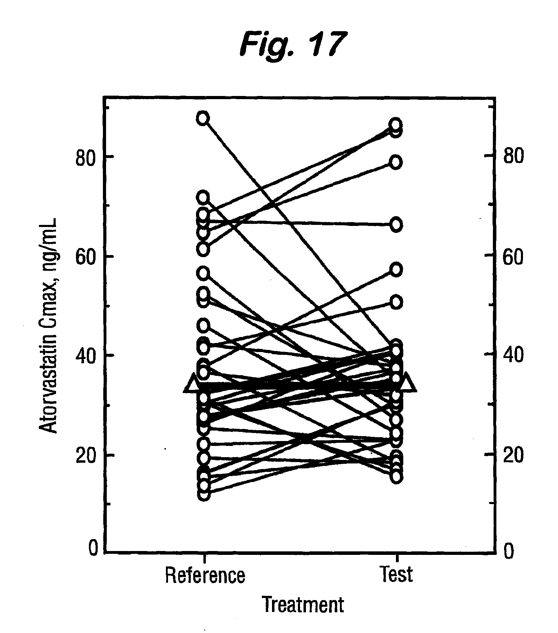

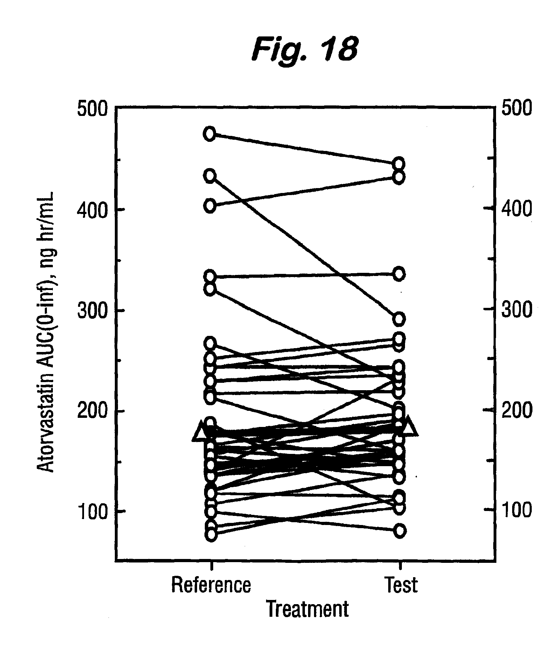

The invention is further described by the following nonlimiting examples which refer to the accompanying Figures 1 to 18, short particulars of which are given below.
Figure 1 Mean amlodipine plasma concentration-time profiles following coadministration of 5-mg amlodipine and 10-mg atorvastatin tablets (closed symbols) and 5-mg amlodipine/10-mg atorvastatin dual therapy tablets (open symbols). Upper and lower panels are linear and semi-logarithmic plots, respectively.
Figure 2 Mean atorvastatin plasma concentration-time profiles following coadministration of 5-mg amlodipine and 10-mg atorvastatin tablets (closed symbols) and 5-mg amlodipine/10-mg atorvastatin dual therapy tablets (open symbols). Upper and lower panels are linear and semi-logarithmic plots, respectively.
Figure 3 Individual amlodipine Cmax values following coadministration of 5-mg amlodipine and 10-mg atorvastatin tablets (Reference) and 5-mg amlodipine/10-mg atorvastatin dual therapy tablets (Test). Individual subject and mean values represented by circles and triangles, respectively.
Figure 4 Individual amlodipine AUC(0-∞) values following coadministration of 5-mg amlodipine and 10-mg atorvastatin tablets (Reference) and 5-mg amlodipine/10-mg atorvastatin dual therapy tablets (Test). Individual subject and mean values represented by circles and triangles, respectively.
Figure 5 Individual atorvastatin Cmax values following coadministration of 5-mg amlodipine and 10-mg atorvastatin tablets (Reference) and 5-mg amlodipine/10-mg atorvastatin dual therapy tablets (Test). Individual subject and mean values represented by circles and triangles, respectively.
Figure 6 Individual atorvastatin AUC(0-∞) values following coadministration of 5-mg amlodipine and 10-mg atorvastatin tablets (Reference) and 5-mg amlodipine/10-mg atorvastatin dual therapy tablets (Test). Individual subject and mean values represented by circles and triangles, respectively.
Figure 7 Mean amlodipine plasma concentration-time profiles following coadministration of 10-mg amlodipine and 40-mg atorvastatin tablets (closed symbols) and 10-mg amlodipine/40-mg atorvastatin dual therapy tablets (open symbols). Upper and lower panels are linear and semilogarithmic plots, respectively.
Figure 8 Mean atorvastatin plasma concentration-time profiles following coadministration of 10-mg amlodipine and 40-mg atorvastatin tablets (closed symbols) and 10-mg amlodipine/40-mg atorvastatin dual therapy tablets (open symbols). Upper and lower panels are linear and semilogarithmic plots, respectively.
Figure 9 Individual amlodipine Cmax values following coadministration of 10-mg amlodipine and 40-mg atorvastatin tablets (Reference) and 10-mg amlodipine/40-mg atorvastatin dual therapy tablets (Test). Individual subject and mean values represented by circles and triangles, respectively.
Figure 10 Individual amlodipine AUC(0-∞) values following coadministration of 10-mg amlodipine and 40-mg atorvastatin tablets (Reference) and 10-mg amlodipine/40-mg atorvastatin dual therapy tablets (Test). Individual subject and mean values represented by circles and triangles, respectively.
Figure 11 Individual atorvastatin Cmax values following coadministration of 10-mg amlodipine and 40-mg atorvastatin tablets (Reference) and 10-mg amlodipine/40-mg atorvastatin.dual therapy tablets (Test). Individual subject and mean values are represented by circles and triangles, respectively.
Figure 12 Individual atorvastatin AUC(0-∞) values following coadministration of 10-mg amlodipine and 40-mg atorvastatin tablets (Reference) and 10-mg amlodipine/40-mg atorvastatin dual therapy tablets (Test). Individual subject and mean values represented by circles and triangles, respectively.
Figure 13 Mean amlodipine plasma concentration-time profiles following coadministration of 10-mg amlodipine and 2 x 40-mg atorvastatin tablets (closed symbols) and 10-mg amlodipine/80-mg atorvastatin dual therapy tablets (open symbols). Upper and lower panels are linear and semilogarithmic plots, respectively.
Figure 14 Mean atorvastatin plasma concentration-time profiles following coadministration of 10-mg amlodipine and 2 x 40-mg atorvastatin tablets (closed symbols) and 10-mg amlodipine/80-mg atorvastatin dual therapy tablets (open symbols). Upper and lower panels are linear and semilogarithmic plots, respectively.
Figure 15 Individual amlodipine Cmax values following coadministration of 10-mg amlodipine and 2 x 40-mg atorvastatin tablets (Reference) and 10-mg amlodipine/80-mg atorvastatin dual therapy tablets (Test). Individual subject and mean values represented by circles and triangles, respectively.
Figure 16 Individual amiodipine AUC(0-∞) values following coadministration of 10-mg amlodipine and 2 x 40-mg atorvastatin tablets (Reference) and 10-mg amlodipine/80-mg atorvastatin dual therapy tablets (Test). Individual subject and mean values represented by circles and triangles, respectively.
Figure 17 Individual atorvastatin Cmax values following coadministration of 10-mg amlodipine and 2 x 40-mg atorvastatin tablets (Reference) and 10-mg amlodipine/80-mg atorvastatin dual therapy tablets (Test). Individual subject and mean values are represented by circles and triangles, respectively.
Figure 18 Individual atorvastatin AUC(0-∞) values following coadministration of 10-mg amlodipine and 2 x 40-mg atorvastatin tablets (Reference) and 10-mg amlodipine/80-mg atorvastatin dual therapy tablets (Test). Individual subject and mean values represented by circles and triangles, respectively.

### DETAILED DESCRIPTION OF THE INVENTION

The pharmaceutical compositions of the present invention comprise amlodipine or a pharmaceutically acceptable acid addition salt thereof and atorvastatin or a pharmaceutically acceptable base addition salt thereof.

Amlodipine may readily be prepared as described in United States Patent Number 4,572,909 which is incorporated herein by reference. Amlodipine besylate, which is currently sold as Norvasc®, may be prepared as described in United States Patent Number 4,879,303 which is incorporated herein by reference. Amlodipine and amlodipine besylate are potent and long-lasting calcium channel blockers.

Atorvastatin may readily be prepared as described in United States Patent Numbers 5,273,995 and 5,969,156 which are incorporated herein by reference. The hemicalcium salt of atorvastatin is currently sold as Lipitor®.

Pharmaceutically acceptable acid addition salts of the compounds of the present invention include salts derived from nontoxic inorganic acids such as hydrochloric, nitric, phosphoric, sulfuric, hydrobromic, hydriodic, hydrofluoric, phosphorous, and the like, as,well as the salts derived from nontoxic organic acids, such as aliphatic mono- and dicarboxylic acids, phenyl-substituted alkanoic acids, hydroxy alkanoic acids, alkanedioic acids, aromatic acids, aliphatic, and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S.M. et al., "Pharmaceutical Salts." J. of Pharma. Sci., 1977;66:1).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge et al., supra., 1977).

The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present invention.

Additionally, the compounds of the present invention can exist in unsolvated forms as well as solvated forms, including hydrated forms. In general, the solvated forms, including hydrated forms, are equivalent to unsolvated forms and are intended to be encompassed within the scope of the present invention.

Amlodipine is a racemic compound due to the symmetry at position 4 of the dihydropyridine ring. The R and S enantiomers may be prepared as described by Arrowsmith et al., J. Med. Chem., 1986;29:1696. The calcium channel blocking activity of amlodipine is substantially confined to the S(-) isomer and to the racemic mixture containing the R(+) and S(-) forms [see International Patent Application Number PCT/EP94/02697 (WO 95/05822)]. The R(+) isomer has little or no calcium channel blocking activity. However, the R(+) isomer is a potent inhibitor of smooth muscle cell migration. Thus, the R(+) isomer is useful in the treatment or prevention of atherosclerosis [see International Patent Application Number PCT/EP95/00847 (WO 95/25722)]. Based on the above, a skilled person could choose the R(+) isomer, the S(-) isomer, or the racemic mixture of the R(+) isomer and the S(-) isomer for use in the combination of this invention.

For preparing pharmaceutical compositions from the compounds of the present invention, pharmaceutically acceptable carriers are solids. Solid form preparations include powders, tablets, pills, capsules, cachets, and suppositories. A solid carrier can be one or more substances which may also act as diluents, flavoring agents, solubilizers, lubricants, suspending agents, binders, preservatives, tablet disintegrating agents, or an encapsulating material.

For example, anionic surfactants include docusate sodium and sodium lauryl sulfate: binders include acacia, carbomer, carboxymethylcellulose sodium, dextrin, ethylcellulose, gelatin, guar gum, hydrogenated vegetable oil (type 1), hydroxyethyl cellulose, hydroxypropyl cellulose, hydroxypropyl methylcellulose, magnesium aluminum silicate, maltodextrin, methylcellulose, polymethacrylates, povidone, pregelatinized starch, sodium alginate, starch, and zein; cationic surfactants include benzalkonium chloride, benzethonium chloride, and certrimide: diluents include calcium carbonate, calcium sulfate, dextrates, dextrin, dextrose, dibasic calcium phosphate dihydrate, glyceryl palmitostearate, hydrogenated vegetable oil (type 1), kaloin, magnesium carbonate, magnesium oxide, maltodextrin, mannitol, microcrystalline cellulose, polymethacrylates, potassium chloride, powdered cellulose, pregelatinized starch, sodium chloride, sorbitol, starch, talc, and tribasic calcium phosphate; disintegrants include carboxymethylcellulose calcium, carboxymethylcellulose sodium, colloidal silicon dioxide, croscarmellose sodium, crospovidone, guar gum, magnesium aluminum silicate, methylcellulose, microcrystalline cellulose, polacrilin potassium, powdered cellulose, pregelatinized starch, sodium alginate, sodium starch glycolate, and starch: flavoring agents include ethyl maltol, ethyl vanillin, maltol, menthol, and vanillin, glidants include colloidal silicon dioxide, magnesium trisilicate, powdered cellulose, starch, talc, and tribasic calcium phosphate: granulating agents include acacia, dextrose, gelatin, povidone, starch, and tragacanth: lubricants include calcium stearate, glyceryl monostearate, glyceryl palmitostearate, hydrogenated caster oil, hydrogenated vegetable oil (type 1), light mineral oil, lubritab, magnesium sterate, mineral oil, polyethylene glycol, sodium benzoate, sodium lauryl sulfate, sodium stearyl fumarate, stearic acid, talc, and zinc stearate; nonionic surfactants include glyceryl monooleate, polyoxyethylene sorbitan fatty acid esters, polyvinyl alcohol, and sorbitan esters: preservatives include alcohol, benzalkonium chloride, benzethonium chloride, benzyl alcohol, bronopol, butylparaben, cetrimide, chlorhexidine, chlorobutanol, chlorocresol, cresol, ethylparaben, glycerin, imidurea, methylparaben, phenol, phenoxyethanol, phenylethyl alcohol, phenylmercuric acetate, phenylmercuric borate, phenylmercuric nitrate, potassium sorbate, propylene glycol, propylparaben, sodium benzoate, sodium propionate, and thimerosal; solubilizing agents include benzalkonium chloride, benzethonium chloride, benzyl benzoate, cyclodextrins, glyceryl monostearate, lecithin, poloxamer, polyoxyethylene alkyl ethers, polyoxyethylene castor oil derivatives, polyoxyethylene sorbitan fatty acid esters, polyoxyethylene stearates, sorbitan esters and stearic acid; suspending agents include acacia, bentonite, carbomer, carboxymethylcellulose calcium, carboxymethylcellulose sodium, colloidal silicon dioxide, dextrin, gelatin, guar gum, hydroxyethyl cellulose, hydroxypropyl cellulose, hydroxypropyl methylcellulose, kaolin, magnesium aluminum silicate, maltitol solution, methylcellulose, microcrystalline cellulose, povidone, powdered cellulose, propylene glycol alginate, sodium alginate, sodium starch glycolate, starch, tragacanth, and xanthan gum.

In powders, the carrier is a finely divided solid which is in a mixture with the finely divided active component.

In solid dosage form, the active component is mixed with the carrier having the necessary binding properties in suitable proportions and compacted in the shape and size desired.

The powders and tablets preferably contain from 5% to about 70% of the active compound. Suitable carriers are magnesium carbonate, magnesium stearate, talc, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component, with or without other carriers, is surrounded by a carrier, which is thus in association with it. Similarly, cachets and lozenges are included. Tablets, powders, capsules, pills, cachets, and lozenges can be used as solid dosage forms suitable for oral administration.

The pharmaceutical preparation is preferably in unit dosage form containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Specifically, the pharmaceutical compositions of the present invention are prepared using the following general procedure:
[A] An atorvastatin granulation is prepared as follows:
   Step (1) - a surface active agent, such as, for example, polysorbate 80, sodium lauryl sulfate, and the like is dissolved in water and a binder, such as, for example, hydroxypropyl cellulose, povidone, hydroxypropylmethyl cellulose (HPMC), Starch 1500, starch, and the like is added and hydrated;
   Step (2) - atorvastatin calcium is mixed with an alkalizing agent that forms a pH greater than 5, such as, for example, calcium carbonate, di- and tri-calcium phosphate and the like, a filler/diluent, such as, for example, microcrystalline cellulose, silicified microcrystalline cellulose, starch. Starch 1551, sorbitol, mannitol, and the like, a filler/diluent/disintegrating agent, such as, for example, Starch 1551, Starch 1550. and the like, and a disintegrating agent, such as, for example, croscarmellose sodium, sodium starch glycolate, polyplasdone, starch, carboxymethyl cellulose (CMC) and the like in a granulating apparatus, such as, for example, a fluid bed granulator/dryer, a high shear mixer/granulator, a twin shell mixer/granulator, a ribbon mixer granulator, and the like;
   Step (3) - the powder mix from Step (2) is granulated with the solution from Step (1) in a granulating apparatus; and
   Step (4) - the granulation is dried in a drying apparatus, such as, for example, a fluid bed granulator/dryer, an oven, a conveyor belt dryer, a microwave dryer, and the like;
[B] A final formulation is prepared as follows:
   Step (1) - amlodipine besylate, a filler/diluent, such as, for example, microcrystalline cellulose, silicified microcrystalline cellulose, starch, Starch 1551, and the like, a disintegrating agent, such as, for example, croscarmellose sodium, sodium starch glycolate, polyplasdone, starch, CMC and the like, and a glidant, such as, for example, silicon dioxide, talc, sterotex, stearic acid, syloid, and the like are added to the atorvastatin granulation and milled by passing through a mill, such as, for example, a Comil mill, a Fritz mill, an Oscillator mill, a Pin mill, and the like;
   Step (2) - the milled material is blended in a blender such as described above with a lubricating agent, such as, for example, magnesium stearate, calcium stearate, zinc stearate, talc, and the like; and
   Step (3) - the blended granulation is compressed in a compressing apparatus into tablets.

Preferably, the granulator dryer used in preparing the pharmaceutical compositions is a Fluid Bed Granulator Dryer (FBGD).

Thus, the pharmaceutical compositions of the present invention contain in addition to the active pharmaceutical agents an alkalizing agent, which is used as a "bioavailability regulator" to control the solubility and bioavailability of the formulation and as a "stability enhancer." The term "bioavailability regulator" means a substance used in the formulation that has an effect on the solubility of the active pharmaceutical agent(s) and thus can be used to regulate the pharmakinetic parameters of the agents. The term "stability enhancer" refers to the use of an alkalizing agent to stabilize atorvastatin or a pharmaceutically acceptable salt thereof in the present pharmaceutical compositions.

"Bioavailability regulators" may be used in a positive sense, that is, their presence may serve to enhance the blood level of the formulation or they may be used in a negative sense where their presence serves to suppress the blood level of the formulation. Thus, it is possible, by using an appropriate amount of a suitable bioavailability regulator, to optimize the bioavailability of a particular formulation.

As indicated, the compositions of the present invention employ as the bioavailability regulator an alkalizing agent, such as calcium carbonate, dicalcium carbonate, tricalcium carbonate, and the like.

In tablets prepared according to the invention, the alkalizing agent behaves in a positive sense and serves to enhance the bioavailability of the atorvastatin component. Preferably, calcium carbonate is used in a ratio of about 1:1 to 1:4 weight/weight (w/w) of atorvastatin calcium to calcium carbonate. Most preferred is a ratio of 1:3 w/w of atorvastatin calcium to calcium carbonate.

Additionally, other preferred carriers include microcrystalline cellulose, Starch 1551, Starch 1500, croscarmellose sodium, polysorbate 80, hydroxypropyl cellulose, silicon dioxide, and magnesium stearate used in the pharmaceutical compositions of the present invention.

The pharmaceutical compositions of the present invention comprise about 0.25% to about 10% amlodipine or a pharmaceutical acceptable salt thereof and about 2.5% to about 20% atorvastatin or a pharmaceutically acceptable salt thereof; preferably about 0.5% to about 7% amlodipine besylate and about 10% to about 20% atorvastatin calcium.

In accordance with the present invention, the following are preferred fixed dual therapy dosage combinations used in the pharmaceutical compositions.

| Atorvastatin calcium (mg), as active | Amlodipine besylate (mg), as active |
|---|---|
| 5 | 2.5 |
| 10 | 2.5 |
| 20 | 2.5 |
| 40 | 2.5 |
| 80 | 2.5 |
| 5 | 5 |
| 10 | 5 |
| 20 | 5 |
| 40 | 5 |
| 80 | 5 |
| 5 | 10 |
| 10 | 10 |
| 20 | 10 |
| 40 | 10 |
| 80 | 10 |

The present invention relates to the treatment of diseases and conditions in a subject, such as, angina pectoris, atherosclerosis, combined hypertension and hyperlipidemia and/or hypercholesterolemia, and to treat subjects presenting with symptoms of cardiac risk with a combination of active ingredients as described above that may be administered in a solid dosage form having low levels of degradation products and/or impurities contained in a therapeutic package or kit. The kit includes the solid dosage form and a container. Typically, the kit includes directions for the administration of the dosage form. The container can be in any conventional shape or form as known in the art, for example, a paper box, a glass or plastic bottle, or a blister pack with individual dosage for pressing out of the back according to a therapeutic schedule.

The pharmaceutical compositions and methods of the present invention are all adapted to therapeutic use as agents in the treatment of angina pectoris, atherosclerosis, and a condition characterized by the presence of both hypertension and hyperlipidemia in mammals, particularly humans. Further, since these diseases and conditions are closely related to the development of cardiac disease and adverse cardiac conditions, these combinations and methods, by virtue of their action as antianginals, antiatherosclerotics, antihypertensives and antihyperlipidemics, are useful in the management of cardiac risk.

Where used herein, the term "cardiac risk" means the likelihood that a subject will suffer a future adverse cardiac event such as. e.g., myocardial infarction, cardiac arrest, cardiac failure, cardiac ischemia. Cardiac risk is calculated using the Framingham Risk Equation as set forth above. The term "cardiac risk management" means that the risk of future adverse cardiac events is substantially reduced.

The utility of the compounds of the present invention as medicinal agents in the treatment of atherosclerosis in mammals (e.g., humans) is demonstrated by the activity of the compounds of the invention in conventional assays and clinical protocols described in International Published Patent Application Number WO 99/11259, which is incorporated herein by reference.

The following dosage amounts and other dosage amounts set forth elsewhere in the specification and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. All doses set forth herein, and in the appendant claims, are daily doses.

In general, in accordance with this invention, amlodipine besylate is generally administered in a dosage of about 0.5 mg to about 20 mg of the active. Preferably, amlodipine besylate is administered in a dosage of about 5 mg to about 10 mg of the active. It will be recognized by a skilled person that the free base form or other salt forms of amlodipine besylate may be used in this invention. Calculation of the dosage amount for these other forms of or the free base form or other salt forms of amlodipine besylate is easily accomplished by performing a simple ratio relative to the molecular weights of the species involved.

In general, in accordance with this invention, atorvastatin is administered in a dosage of about 0.5 mg to about 160 mg of the active. Preferably, atorvastatin is administered in a dosage of about 10 mg to about 80 mg of the active. It will be recognized by a skilled person that the free acid form or other salt forms of atorvastatin calcium may be used in this invention. Calculation of the dosage amount for these other forms of or the free acid form or other salt forms of atorvastatin calcium is easily accomplished by performing a simple ratio relative to the molecular weights of the species involved.

### BIOEQUIVALENCE STUDIES

Single-dose bioequivalence studies were carried out comparing amlodipine besylate/atorvastatin calcium dual therapy tablets to coadministered amlodipine besylate and atorvastatin calcium tablets.

Specifically, comparisons were carried out between the following dosage regimens:
(1) 5-mg amlodipine/10-mg atorvastatin dual therapy tablet versus 5-mg amlodipine and 10-mg atorvastatin tablets
(2) 10-mg amlodipine/40-mg atorvastatin dual therapy tablet versus 10-mg amlodipine and 40-mg atorvastatin tablets
(3) 10-mg amlodipine/80-mg atorvastatin dual therapy tablet versus 10-mg amlodipine and two 40-mg atorvastatin tablets

In all cases, the dual therapy tablets were bioequivalent to coadministration of separate amlodipine and atorvastatin tablets. The details of the studies are described in Examples 2-4 and Tables 1-3.

### STABILITY STUDIES

Total impurities and/or degradants from atorvastatin after storage of the pharmaceutical composition at 25°C/60% relative humidity (RH) for 24 months should not be more than 2.0%. Additionally, the following specific impurities and/or degradants should not be more than 0.5%:
5-(4-Fluorophenyl)-2,3-dihydro-β,δ-dihydroxy-3-(1-methylethyl)-2-oxo-4-phenyl-3-[(phenylamino)carbonyl]-1 H-pyrrole-1-heptanoic acid;
(2R-trans)-5-(4-Fluorophenyl)-2-(1-methylethyl)-N,4-diphenyl-1-[2-(tetrahydro-4-hydroxy-6-oxo-2H-pyran-2-yl)ethyl]-1H-pyrrole-3-carboxamide; and
3-[(4-Fluorophenyl)carbonyl]-2-(2-methyl-1-oxopropyl)-N.3-diphenyl-2-oxiranecarboxamide.

Total impurities and/or degradants from amlodipine after storage of the pharmaceutical composition at 25°C/60% RH for 24 months should not be more than 2.0%. Additionally, the following specific impurities and/or degradants should not be more than 1.0%:
2-(2-Amino-ethoxymethyl)-4-(2-chloro-phenyl)-6-methyl-pyridine-3.5-dicarboxylic acid 3-ethyl ester 5-methyl ester; and
6-(2-Chloro-phenyl)-8-methyl-3.4.6,7-tetrahydro-2H-1,4-benzoxazine-5.7-dicarboxylic acid 5-ethyl ester 7-methyl ester.

The stability of atorvastatin/amlodipine dual therapy tablets stored at 40°C/75% RH were evaluated. Specifically, the following combinations were evaluated:
(1) 5mg amlodipine/10 mg atorvastatin
(2) 10 mg amlodipine/40 mg atorvastatin
(3) 10 mg amlodipine/80 mg atorvastatin

Table 4 shows the results of analysis for degradation products of the dual therapy tablets compared to commercial Lipitor® tablets (atorvastatin calcium) after 3-month stability at 40°C/75% RH. In all cases, the total degradation products of the dual therapy tablets were comparable to or better than those for the Lipitor® tablets.

This accelerated study at 40°C/75% RH for 3 months is a standard procedure for predicting shelf life stability of pharmaceuticals at 25°C/60% RH for 24 months.

The above results show that the pharmaceutical compositions of the present invention are not only stable but also have bioavailability equivalent to administering each of the therapeutic agents in a separate dosage form.

The following nonlimiting examples illustrate the inventors' preferred methods for preparing and using the pharmaceutical compositions of the present invention.

**Table 1. Summary of Pharmacokinetic Parameter Values Following Coadministration of 5-mg Amlodipine and 10-mg Atorvastatin Tablets (Reference) and 5-mg Amlodipine/10-mg Atorvastatin Dual Therapy Tablets (Test)**

| Parameter | Least-Squares Mean Values | | Ratio | 90% Confidence Interval |
|---|---|---|---|---|
| | Coadministration Separate Tablets (Reference) | Dual Therapy Tablets (Test) | | |
| Amlodipine, Standard Analysis | | | | |
| Cmax, ng/mL | 2.79 | 2.77 | 99.1 | 95.7 to 103 |
| tmax, hr | 7.41 | 8.06 | 109 | Not Applicable |
| AUC(0-tlqc), ng.hr/mL | 136 | 134 | 98.1 | 94.9 to 101 |
| AUC(0-∞), ng.hr/mL | 152 | 149 | 98.2 | 94.8 to 102 |
| t½, hr | 51.6 | 49.5 | 96.1 | 88.8 to 103 |

| Amlodipine, Normalized for Content | | | | |
|---|---|---|---|---|
| nCmax. ng/mL | 2.79 | 2.94 | 105 | 102 to 109 |
| nAUC(0-tlqc), ng.hr/mL | 136 | 142 | 104 | 101 to 108 |
| nAUC(0-∞). ng.hr/mL | 152 | 159 | 104 | 101 to 108 |

| Atorvastatin | | | | |
|---|---|---|---|---|
| Cmax.ng/mL | 2.52 | 2.30 | 91.0 | 82.0 to 101 |
| tmax.hr | 0.624 | 1.12 | 180 | Not Applicable |
| AUC(0-tlqc). ng.hr/mL | 12.8 | 12.3 | 95.8 | 88.6 to 104 |
| AUC(0-∞), ng·hr/mL | 18.4 | 18.4 | 100 | 90.2 to 111 |
| t½. hr | 9.12 | 10.2 | 112 | 82.0 to 142 |

| | | | | |
|---|---|---|---|---|
| AUC(O-∞) = Area under plasma concentration-time profile from time zero extrapolated to infinity. t½ = Terminal half-life. nCmax and nAUC = Values normalized for amlodipine content. Ratio = Ratio of treatment mean values, expressed as a percentage (100% x test/reference). 90% Confidence Interval = 90% confidence interval estimate for the ratio (test/reference) of treatment mean values, expressed as a percentage of the reference mean. | | | | |

**Table 2. Summary of Pharmacokinetic Parameter Values Following Coadministration of 10-mg Amlodipine and 40-mg Atorvastatin Tablets (Reference) and 10-mg Amlodipine/40-mg Atorvastatin Dual Therapy Tablets (Test)**

| Parameter | Least-Squares Mean Values | | Ratio | 90% Confidence Interval |
|---|---|---|---|---|
| | Coadministration Separate Tablets (Reference) | Dual Therapy Tablets (Test) | | |
| Amlodipine | | | | |
| Cmax, ng/mL | 5.77 | 6.26 | 109 | 105 to 113 |
| tmax, hr | 7.28 | 7.33 | 101 | Not Applicable |
| AUC(0-tlqc), ng·hr/mL | 287 | 298 | 104 | 101 to 107 |
| AUC(0-∞), ng·hr/mL | 320 | 331 | 103 | 100 to 107 |
| t½, hr | 51.7 | 51.6 | 99.7 | 94.6 to 105 |

| Atorvastatin | | | | |
|---|---|---|---|---|
| Cmax, ng/mL | 15.0 | 14.2 | 95.0 | 82.1 to 110 |
| tmax, hr | 0.641 | 1.09 | 170 | Not Applicable |
| AUC(0-tlqc), ng·hr/mL | 71.5 | 79.1 | 111 | 104 to 117 |
| AUC(O-∞), ng·hr/mL | 80.4 | 88.2 | 110 | 103 to 116 |
| t½, hr | 12.3 | 15.3 | 124 | 98.2 to 149 |

| | | | | |
|---|---|---|---|---|
| Cmax = Maximum plasma concentration. tmax = Time for Cmax. AUC(0-tlqc) = Area under plasma concentration-time profile from time zero to time for the last quantifiable concentration. AUC(0-∞) = Area under plasma concentration-time profile from time zero extrapolated to infinity. t½ = Terminal half-life. Ratio = Ratio of treatment mean values, expressed as a percentage (100% x test/reference). 90% Confidence Interval = 90% confidence interval estimate for the ratio (test/reference) of treatment mean values, expressed as a percentage of the reference mean. | | | | |

**Table 3. Summary of Pharmacokinetic Parameter Values Following Coadministration of 10-mg Amlodipine and 2 x 40-mg Atorvastatin Tablets (Reference) and 10-mg Amlodipine/80-mg Atorvastatin Dual Therapy Tablets (Test)**

| Parameter | Least-Squares Mean Values | | Ratio | 90% Confidence Interval |
|---|---|---|---|---|
| | Coadministration Separate Tablets (Reference) | Dual Therapy Tablets (Test) | | |
| Amlodipine | | | | |
| Cmax, ng/mL | 5.08 | 5.00 | 98.6 | 95.4 to 102 |
| tmax, hr | 7.39 | 7.44 | 101 | Not Applicable |
| AUC(0-tlqc), ng·hr/mL | 270 | 262 | 97.0 | 94.2 to 99.9 |
| AUC(0-∞), ng·hr/mL | 303 | 298 | 98.4 | 95.4 to 101 |
| t½ hr | 52.6 | 55.7 | 106 | 99.9 to 112 |

| Atorvastatin | | | | |
|---|---|---|---|---|
| Cmax, ng/mL | 33.7 | 33.7 | 100 | 87.8 to 114 |
| tmax, hr | 1.09 | 1.58 | 144 | Not Applicable |
| AUC(0-tlqc), ng·hr/mL | 168 | 170 | 101 | 95.1 to 108 |
| AUC(0-∞),ng·hr/mL | 177 | 181 | 95 | 94.8 to 109 |
| t½, hr | 12.7 | 15.51 | 122 | 101 to 143 |

| | | | | |
|---|---|---|---|---|
| Cmax = Maximum plasma concentration. tmax = Time for Cmax. AUC(0-tlqc) = Area under plasma concentration-time profile from time zero to time for the last quantifiable concentration. AUC(0-∞) = Area under plasma concentration-time profile from time zero extrapolated to infinity. t½ = Terminal half-life. Ratio = Ratio of treatment mean values, expressed as a percentage (100% x test/reference). 90% Confidence Interval = 90% confidence interval estimate for the ratio (test/reference) of treatment mean values, expressed as a percentage of the reference mean. | | | | |

**Table 4. Comparative Stability Results of Amlodipine/Atorvastatin Dual Therapy Tablets and Lipitor® Tablets**

| Degradation of Tablets Stored at 40°C/75% R11 for 3 Months | | | | | | | | | |
|---|---|---|---|---|---|---|---|---|---|
| Product | Amlodipine/Atorvastatin Dual Therapy Tablets | | | | | | Lipitor® Tablets | | |
| Dose | 5mg/10 mg | | 10 mg/40 mg | | 10 mg/80 mg | | 10 mg | 40 mg | 80 mg |
| Package | Bottle | Blister | Bottle | Blister | Bottle | Blister | Bottle | Bottle | Bottle |
| ***Atorvastatin*** | | | | | | | | | |
| Total Degradation Products (%) | 0.39 | 0.41 | 0.23 | 0.24 | 0.24 | 0.33 | 0.43-0.54 | 0.51-0.63 | 0.20 |

| ***Amlodipine*** | | | | | | | | | |
|---|---|---|---|---|---|---|---|---|---|
| Total Degradation Products (%). | ND | ND | ND | ND | ND | ND | N/A | N/A | N/A |

| | | | | | | | | | |
|---|---|---|---|---|---|---|---|---|---|
| N/A = Not Applicable. ND = None Detected. | | | | | | | | | |

### EXAMPLE 1

### GENERAL PROCESS FOR PREPARING ATORVASTATIN CALCIUM/ AMLODIPINE BESYLATE DUAL THERAPY TABLETS

### [A] Atorvastatin Granulation

Step 1. - Dissolve polysorbate 80 in purified water at 50°C and add and hydrate hydroxypropyl cellulose. Allow the solution to cool to room temperature.
Step 2. - Mix atorvastatin calcium, calcium carbonate, microcrystalline cellulose, starch 1500, and croscarmellose sodium in a Fluid Bed Granulator/Dryer (FBG/D) or a high shear mixer/granulator.
Step 3. - Granulate the powder mix from Step 2 with the solution from Step 1 in the FBG/D or a high shear mixer/granulator.
Step 4. - Dry the granulation in the FBG/D or other drying apparatus to a moisture content (loss on drying, LOD) of less than or equal to 2.0%.

### [B] Final Formulation

Step 1. - Add amlodipine besylate, microcrystalline cellulose, croscarmellose sodium, and silicon dioxide to the atorvastatin granulation from Step [A].
Step 2. - Pass the powder mixture through a mill. e.g.. a Comil mill.
Step 3. - Add magnesium stearate to the milled powder mixture from Step 2 and blend in either a bin blender, a V-blender, a ribbon blender, and the like.
Step 4. - Compress the final blended granulation into tablets using a tableting apparatus.

Table 5. Provides the formulation presentation of amlodipine besylate/atorvastatin calcium dual therapy tablet cores.

**Table 5. Amlodipme/Atorvastatin Dual Therapy Tablet Cores (g/1000 tablets)**

| Atorvastutin Dose (mg) | 10 | | 20 | | 40 | | 80 | |
|---|---|---|---|---|---|---|---|---|
| Amlodipine Dose (mg) | 5 | 10 | 5 | 10 | 5 | 10 | 5 | 10 |
| **Atorvastatin Granulation** | | | | | | | | |
| Atorvaslatin Calcium | 10.85 | 10.85 | 21.70 | 21.70 | 43.40 | 43.40 | 86.80 | 86.80 |
| Calcium Carbonate | 33.1 5 | 33.1 5 | 66.30 | 66.30 | 132.60 | 132.60 | 265.20 | 265.20 |
| Croscarmellose Sodium | 3.00 | 3.00 | 6.00 | 6.00 | 12.00 | 12.00 | 24.00 | 24.00 |
| Microcrystalline Cellulose | 13.85 | 13.85 | 27.70 | 27.70 | 55.40 | 55.40 | 110.80 | 110.80 |
| Starch, Pregelatinized, 1500 Corn | 15.00 | 15.00 | 30.00 | 30.00 | 60.00 | 60.00 | 120.00 | 120.00 |
| Polysorbate 80 | 0.40 | 0.40 | 0.80 | 0.80 | 1.60 | 1.60 | 3.20 | 3.20 |
| Hydroxypropyl Cellulose | 2.00 | 2.00 | 4.00 | 4.00 | 12.00 | 12.00 | 24.00 | 24.00 |
| Purified Water USP/Ep^{a} | 60.00 | 60.00 | 120.00 | 120.00 | 240.00 | 240.00 | 480.00 | 480.00 |

| **Final Blend** | | | | | | | | |
|---|---|---|---|---|---|---|---|---|
| Amlodipine Besylate | 6.94 | 13.87 | 6.94 | 13.87 | 6.94 | 13.87 | 6.94 | 13.87 |
| Microcrystalline Cellulose | 10.41 | 3.48 | 27.76 | 20.83 | 62.46 | 55.53 | 131.86 | 124.93 |
| Croscarmellose Sodium | 3.00 | 3.00 | 6.00 | 6.00 | 12.00 | 12.00 | 24.00 | 24.00 |
| Silicon Dioxide, Colloidal | 0.65 | 0.65 | 1.30 | 1.30 | 2.60 | 2.60 | 5.20 | 5.20 |
| Magnesium Stearate (non-bovine) | 0.75 | 0.75 | 1.50 | 1.50 | 3.00 | 3.00 | 6.00 | 6.00 |
| ***Tablet* Co*re Weight (mg)*** | ***100*** | ***100*** | ***200*** | ***200*** | ***400*** | ***400*** | ***800*** | ***800*** |

| | | | | | | | | |
|---|---|---|---|---|---|---|---|---|
| Formulation aid which is removed during processing | | | | | | | | |

### EXAMPLE 2

### SINGLE-DOSE BIOEQUIVALENCE STUDY COMPARING A 5-MG AMLODIPINE/10-MG ATORVASTATIN DUAL THERAPY TABLET TO COADMINISTERED 5-MG AMLODIPINE AND 10-MG ATORVASTATIN TABLETS

**PROTOCOL:** A randomized, single-dose, 2-way crossover study was carried out in 36 healthy volunteers. Following an overnight fast, each subject received a single 5-mg amlodipine and 10-mg atorvastatin dose as a dual therapy tablet and coadministration of separate tablets on Days 1 and 15.

Blood samples were collected before and serially for 168 hours following each dose. Plasma samples were harvested and stored frozen at -70°C prior to assay. Plasma amlodipine and atorvastatin concentrations were assayed by validated methods. Pharmacokinetic parameter values were evaluated from concentration-time profiles by noncompartmental methods. Results of ANOVA (analysis of variance) of log-transformed Cmax and AUC values were used to calculate 90% confidence intervals for the ratios of least-squares treatment mean values. Bioequivalence would be declared if the confidence intervals for the ratios of amlodipine and atorvastatin Cmax and AUC values, based on log-transformed data, were within the 80% to 125% range.

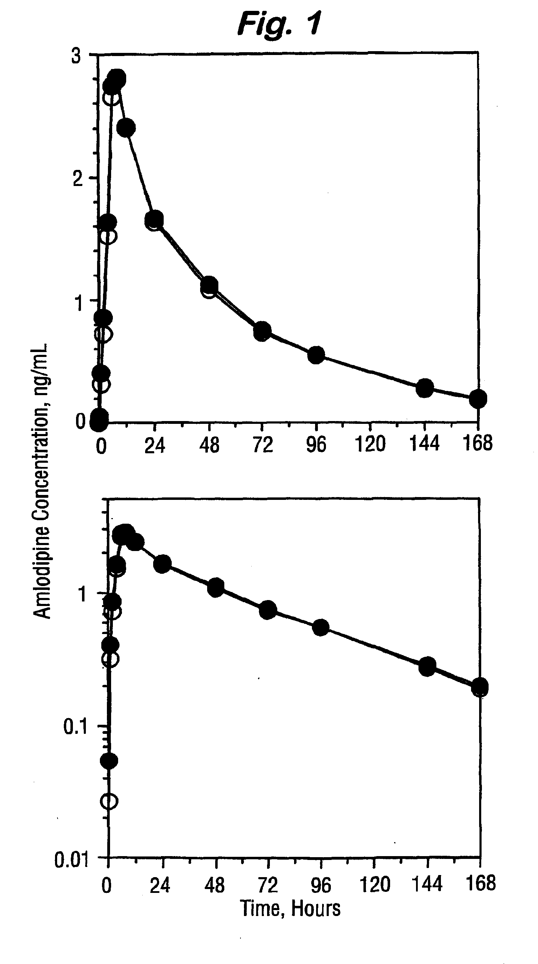

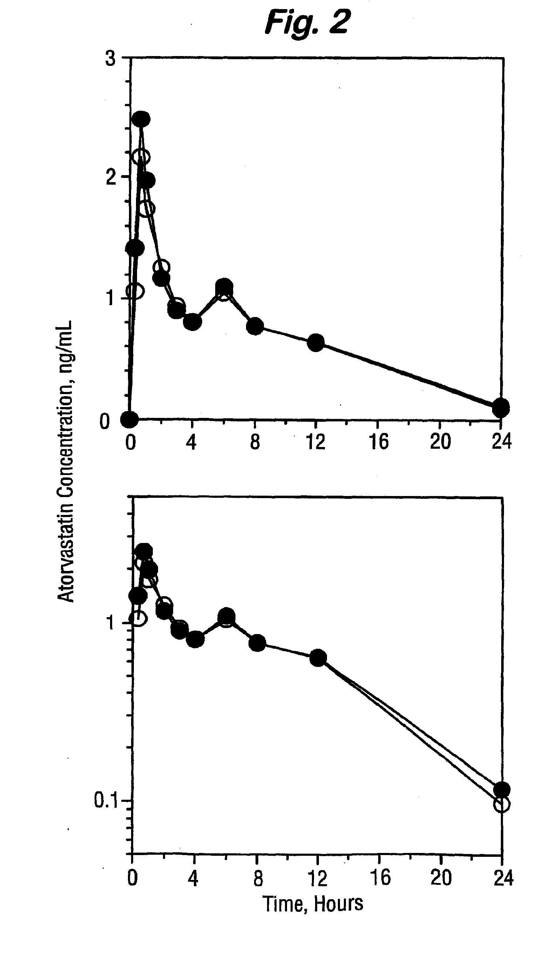

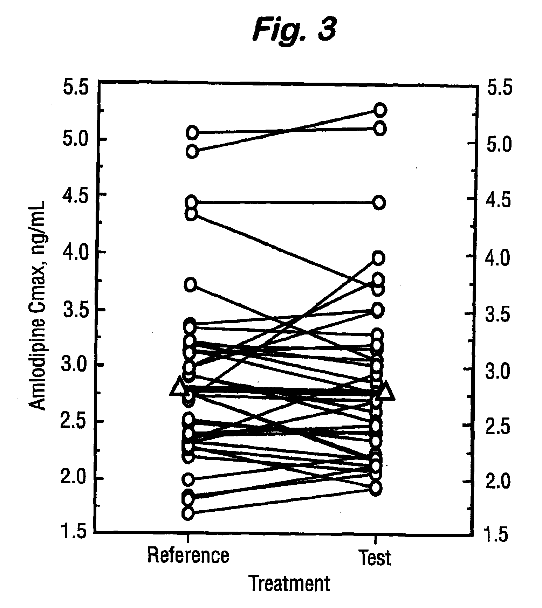

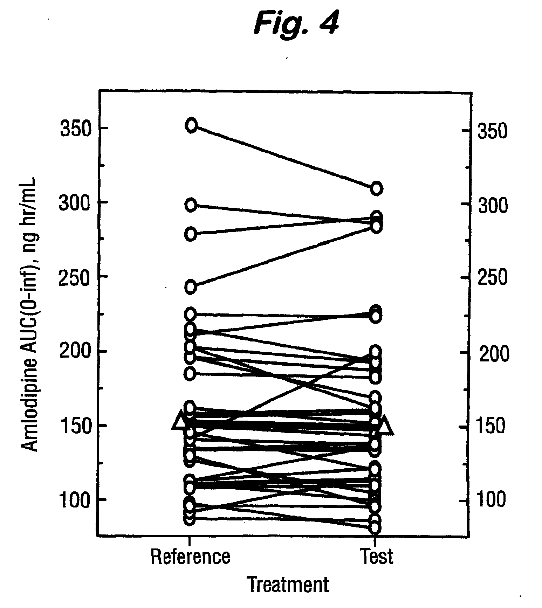

Examination of assay and content uniformity of the dual therapy tablet evaluated in this study revealed that the amlodipine portion was 94% of label claim. The atorvastatin portion was within the 95% to 105% range as were the marketed amlodipine tablets and atorvastatin tablets coadministered in the reference treatment. Therefore, bioequivalence was re-evaluated after dividing amlodipine Cmax and AUC values for the test treatment by 0.94. Results of both analyses are presented.
RESULTS: Data obtained from 35 subjects who completed the study, as well as from one subject who received only the separate tablet treatment before withdrawing from the study, were used in evaluation. Mean plasma concentrations are illustrated in Figures 1 and 2. Pharmacokinetic parameter values are summarized in Table 1. Individual Cmax and AUC values are illustrated in Figures 3 and 4.

### Amlodipine, Standard Analysis

Based on amlodipine Cmax and tmax values, the rate of absorption following administration of 5-mg amlodipine/10-mg atorvastatin dual therapy tablets was similar to that following coadministration of separate 5-mg amlodipine and 10-mg atorvastatin tablets. The difference in mean tmax values was approximately 40 minutes. Mean Cmax values following administration of each treatment were nearly identical, and the 90% confidence interval for Cmax values was within the 80% to 125% bioequivalence range.

Based on amlodipine AUC values, the extent of absorption following administration of 5-mg amlodipine/10-mg atorvastatin dual therapy tablets was similar to that following coadministration of separate 5-mg amlodipine and 10-mg atorvastatin tablets. Mean AUC(0-∞) values were nearly identical, and the 90% confidence interval for AUC(0-∞) values was within the 80% to 125% bioequivalence range.

Mean amlodipine terminal elimination t½ values were similar, averaging approximately 50 hours.

### Analysis Normalized for Amlodipine Content in Test Tablets

The mean amlodipine content-normalized Cmax value following administration of test tablets was approximately 5% higher than that of coadministration of individual tablets. The 90% confidence interval for normalized-Cmax values was within the 80% to 125% bioequivalence range.

The mean amlodipine content-normalized AUC(0-∞) value following administration of test tablets was approximately 4% higher than that of coadministration of individual tablets. The 90% confidence interval for normalized-AUC(0-∞) values was within the 80% to 125% bioequivalence range.

### Atorvastatin

Based on atorvastatin Cmax and tmax values, the rate of absorption following administration of 5-mg amlodipine/10-mg atorvastatin dual therapy tablets was similar to that following coadministration of separate 5-mg amlodipine and 10-mg atorvastatin tablets. The difference in mean tmax values was approximately 30 minutes. The difference in mean Cmax values was approximately 9%, and the 90% confidence interval for Cmax values was within the 80% to 125% bioequivalence range.

Based on atorvastatin AUC values, the extent of absorption following administration of 5-mg amlodipine/10-mg atorvastatin dual therapy tablets was similar to that following coadministration of separate 5-mg amlodipine and 10-mg atorvastatin tablets. Mean AUC(0-∞) values were identical, and the 90% confidence interval for AUC(0-∞) values was within the 80% to 125% bioequivalence range.

Mean atorvastatin terminal elimination t½ values were similar, averaging approximately 10 hours.
**CONCLUSION:** Amlodipine 5-mg/atorvastatin 10-mg dual therapy tablets are bioequivalent to coadministration of separate 5-mg amlodipine and 10-mg atorvastatin tablets.

### EXAMPLE 3

### SINGLE-DOSE BIOEQUIVALENCE STUDY COMPARING A 10-MG AMLODIPINE/40-MG ATORVASTATIN DUAL THERAPY TABLET TO COADMINISTERED 10-MG AMLODIPINE AND 40-MG ATORVASTATIN TABLETS

**PROTOCOL:** A randomized, single-dose, 2-way crossover study was carried out in 36 healthy volunteers. Following an overnight fast, each subject received a single 10-mg amlodipine and 40-mg atorvastatin dose as a dual therapy tablet and coadministration of separate tablets on Days 1 and 15.

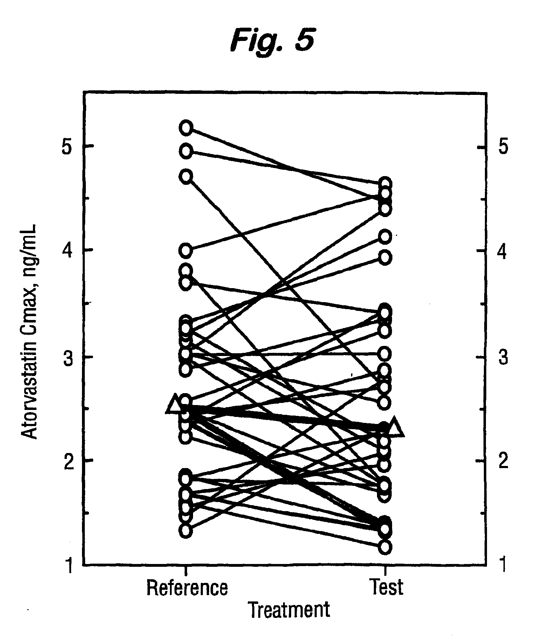

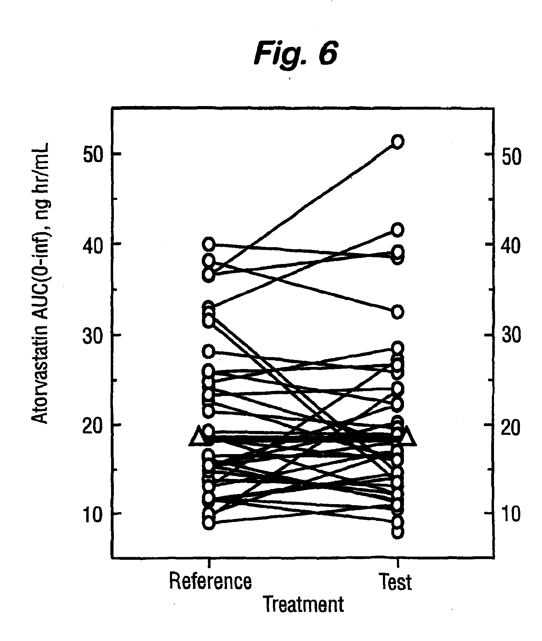

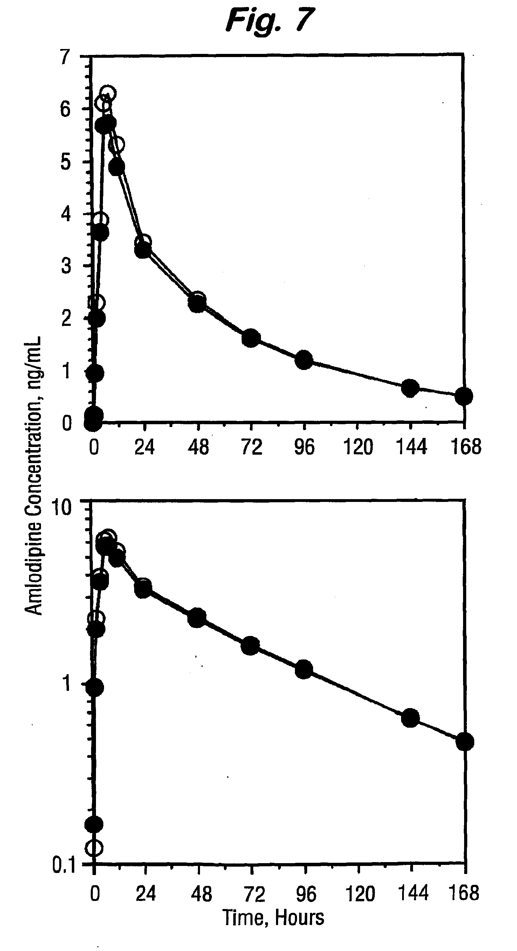

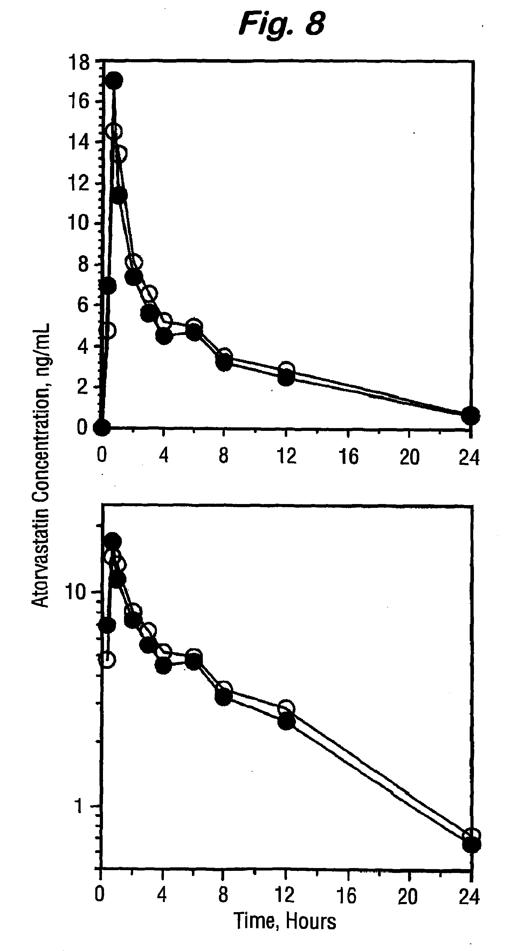

Blood samples were collected before and serially for 168 hours following each dose. Plasma samples were harvested and stored frozen at -70°C prior to assay. Plasma amlodipine and atorvastatin concentrations were assayed by validated methods. Pharmacokinetic parameter values were evaluated from concentration-time profiles by noncompartmental methods. Results of ANOVA of log-transformed Cmax and AUC values were used to calculate 90% confidence intervals for the ratios of least-squares treatment mean values. Bioequivalence would be declared if the confidence intervals for the ratios of amlodipine and atorvastatin Cmax and AUC values, based on log-transformed data, were within the 80% to 125% range.
**RESULTS:** Data obtained from 36 subjects who completed the study were evaluated. Mean plasma concentrations are illustrated in Figures 5 and 6. Pharmacokinetic parameter values are summarized in Table 2. Individual Cmax and AUC values are illustrated in Figures 7 and 8.

### Amlodipine

Based on amlodipine Cmax and tmax values, the rate of absorption following administration of 10-mg amlodipine/40-mg atorvastatin dual therapy tablets was similar to that following coadministration of separate 10-mg amlodipine and 40-mg atorvastatin tablets. The difference in mean tmax values was less than 10 minutes, and the difference in mean Cmax values was 9%. The 90% confidence interval for Cmax values was within the 80% to 125% bioequivalence range.

Based on amlodipine AUC values, the extent of absorption following administration of 10-mg amlodipine/40-mg atorvastatin dual therapy tablets was similar to that following coadministration of separate 10-mg amlodipine and 40-mg atorvastatin tablets. The difference in mean AUC(0-∞) values was 3%, and the 90% confidence interval for AUC(0-∞) values was within the 80% to 125% bioequivalence range.

Mean amlodipine terminal elimination t½ values were similar, averaging approximately 51 hours.

### Atorvastatin

Based on atorvastatin Cmax and tmax values, the rate of absorption following administration of 10-mg amlodipine/40-mg atorvastatin dual therapy tablets was similar to that following coadministration of separate 10-mg amlodipine and 40-ing atorvastatin tablets. The difference in mean tmax values was less than 30 minutes. The difference in mean Cmax values was 5%, and the 90% confidence interval for Cmax values was within the 80% to 125% bioequivalence range.

Based on atorvastatin AUC values, the extent of absorption following administration of 10-mg amlodipine/40-mg atorvastatin dual therapy tablets was similar to that following coadministration of separate 10-mg amlodipine and 40-mg atorvastatin tablets. The difference in mean AUC(0-∞) values was 10%, and the 90% confidence interval for AUC(0-∞) values was within the 80% to 125% bioequivalence range.

Mean atorvastatin terminal elimination t½ values were similar, averaging approximately 14 hours.
**CONCLUSION:** Amlodipine 10-mg/atorvastatin 40-mg dual therapy tablets are bioequivalent to coadministration of separate 10-mg amlodipine and 40-mg atorvastatin tablets.

### EXAMPLE 4

### SINGLE-DOSE BIOEQUIVALENCE STUDY COMPARING A 10-MG AMLODIPINE/80-MG ATORVASTATIN DUAL THERAPY TABLET TO COADMINISTERED 10-MG AMLODIPINE AND TWO 40-MG ATORVASTATIN TABLETS

**PROTOCOL:** A randomized, single-dose. 2-way crossover study was carried out in 36 healthy volunteers. Following an overnight fast, each subject received a single 10-mg amlodipine and 80-mg atorvastatin dose as a dual therapy tablet and coadministration of separate tablets on Days 1 and 15.

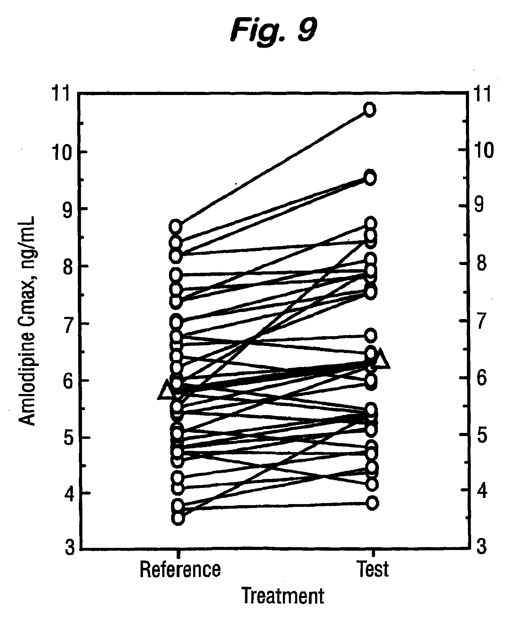

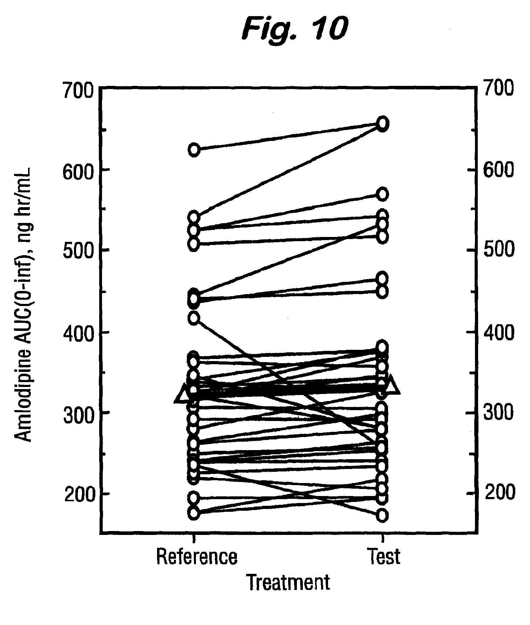

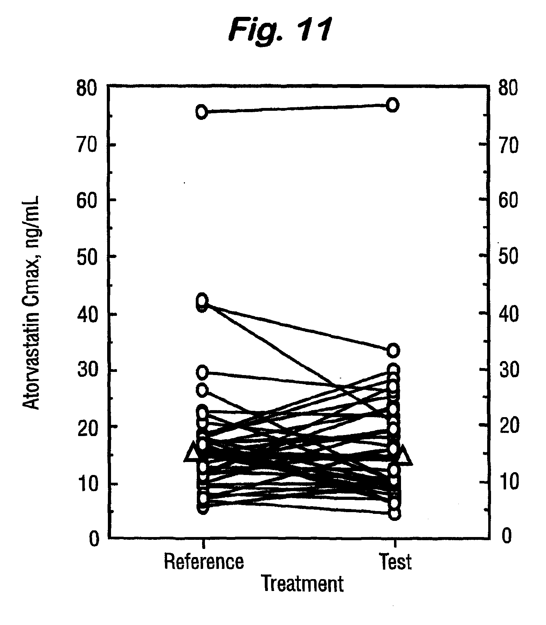

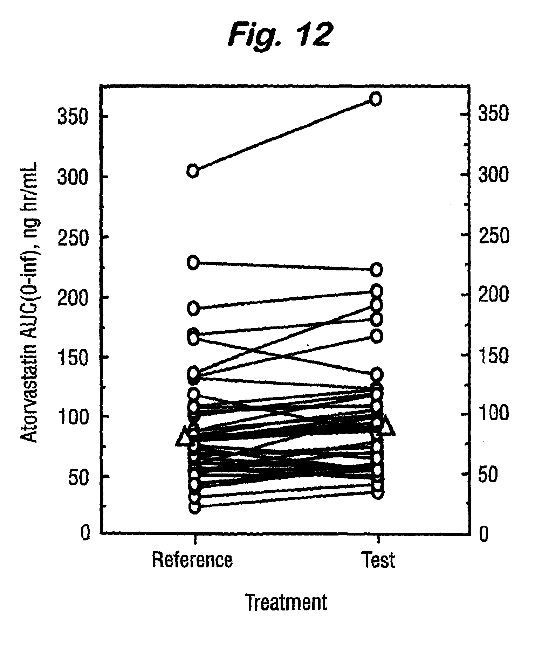

Blood samples were collected before and serially for 168 hours following each dose. Plasma samples were harvested and stored frozen at -70°C prior to assay.,Plasma amlodipine and atorvastatin concentrations were assayed by validated methods. Pharmacokinetic parameter values were evaluated from concentration-time profiles by noncompartmental methods. Results of ANOVA of log-transformed Cmax and AUC values were used to calculate 90% confidence intervals for the ratios of least-squares treatment mean values. Bioequivalence would be declared if the confidence intervals for the ratios of amlodipine and atorvastatin Cmax and AUC values, based on log-transformed data, were within the 80% to 125% range.
**RESULTS:** Data obtained from 36 subjects who completed the study were evaluated. Mean plasma concentrations are illustrated in Figures 9 and 10. Pharmacokinetic parameter values are summarized in Table 3. Individual Cmax and AUC values are illustrated in Figures 11 and 12.

### Amlodipine

Based on amlodipine Cmax and tmax values, the rate of absorption following administration of 10-mg amlodipine/80-mg atorvastatin dual therapy tablets was similar to that following coadministration of separate 10-mg amlodipine and two 40-mg atorvastatin tablets. The difference in mean tmax values was less than 5 minutes, and the difference in mean Cmax values was less than 2%. The 90% confidence interval for Cmax values was within the 80% to 125% bioequivalence range.

Based on amlodipine AUC values, the extent of absorption following administration of 10-mg amlodipine/80-mg atorvastatin dual therapy tablets was similar to that following coadministration of separate 10-mg amlodipine and two 40-mg atorvastatin tablets. The difference in mean AUC(0-∞) values was less than 2%, and the 90% confidence interval for AUC(0-∞) values was within the 80% to 125% bioequivalence range.

Mean amlodipine terminal elimination t½ values were similar, averaging approximately 54 hours.

### Atorvastatin

Based on atorvastatin Cmax and tmax values, the rate of absorption following administration of 10-mg amlodipine/80-mg atorvastatin dual therapy tablets was similar to that following coadministration of separate 10-mg amlodipine and two 40-mg atorvastatin tablets. The difference in mean tmax values was less than 30 minutes. Mean Cmax values were idefitical. The 90% confidence interval for Cmax values was within the 80% to 125% bioequivalence range.

Based on atorvastatin AUC values, the extent of absorption following administration of 10-mg amlodipine/80-mg atorvastatin dual therapy tablets was similar to that following coadministration of separate 10-mg amlodipine and two 40-mg atoryastatin tablets. The difference in mean AUC(0-∞) values was 2%, and the 90% confidence interval for AUC(0-∞) values was within the 80% to 125% bioequivalence range.

Mean atorvastatin terminal elimination t½ values were similar, averaging approximately 14 hours.
**CONCLUSION:** Amlodipine 10-mg/atorvastatin 80-mg dual therapy tablets are bioequivalent to coadministration of separate 10-mg amlodipine and two 40-mg atorvastatin tablets.

## Claims

1. A pharmaceutical composition comprising two components:
(a) one component comprising a granulation of atorvastatin or pharmaceutically acceptable salts thereof and a carrier including an alkalizing agent that forms a pH greater than 5; and
(b) a second component comprising amlodipine or pharmaceutically acceptable salts thereof and a carrier excluding an alkalizing agent that forms a pH greater than 5;
wherein the two components are combined to form a final composition for a solid dosage form.

2. The pharmaceutical composition according to claim 1 comprising about 0.25% to about 10% amlodipine or a pharmaceutically acceptable salt thereof and about 2.5% to about 20% atorvastatin or a pharmaceutically acceptable salt thereof.

3. The pharmaceutical composition according to claim 1 comprising about 0.5 to about 20 mg of amlodipine or a pharmaceutically acceptable salt thereof and about 0.5 to about 160mg of atorvastatin or a pharmaceutically acceptable salt thereof.

4. The pharmaceutical composition according to claim 1 comprising amlodipine besylate and atorvastatin calcium.

5. The pharmaceutical composition according to claim 1 comprising a fixed combination selected from the group consisting of:
atorvastatin calcium, 5 mg active and amlodipine besylate, 2.5 mg active; atorvastatin calcium, 10 mg active and amlodipine besylate, 2.5 mg active; atorvastatin calcium, 20 mg active and amlodipine besylate, 2.5 mg active; atorvastatin calcium, 40 mg active and amlodipine besylate, 2.5 mg active; atorvastatin calcium, 80 mg active and amlodipine besylate, 2.5 mg active; atorvastatin calcium, 5 mg active and amlodipine besylate, 5 mg active; atorvastatin calcium, 10 mg active and amlodipine besylate, 5 mg active; atorvastatin calcium, 20 mg active and amlodipine besylate, 5 mg active; atorvastatin calcium, 40 mg active and amlodipine besylate, 5 mg active; atorvastatin calcium, 80 mg active and amlodipine besylate, 5 mg active; atorvastatin calcium, 5 mg active and amlodipine besylate, 10 mg active; atorvastatin calcium, 10 mg active and amlodipine besylate, 10 mg active; atorvastatin calcium, 20 mg active and amlodipine besylate, 10 mg active; atorvastatin calcium, 40 mg active and amlodipine besylate, 10 mg active; and atorvastatin calcium, 80 mg active and amlodipine besylate, 2.5 mg active.

6. A pharmaceutical composition according to claim 1 comprising amlodipine or pharmaceutically acceptable salts thereof and atorvastatin or pharmaceutically acceptable salts thereof and a carrier containing not more than 2% total impurities and/or degradants from atorvastatin and not more than 2% total impurities and/or degradants from amlodipine after storage at 25°C/60% relative humidity for 24 months.

7. A pharmaceutical composition according to claim 1 comprising amlodipine or pharmaceutically acceptable salts thereof and atorvastatin or pharmaceutically acceptable salts thereof and a carrier containing not more than 0.5% of a compound selected from the group consisting of:
5-(4-Fluorophenyl)-2,3-dihydro-β,δ-dihydroxy-3-(1-methylethyl)-2-oxo-4-phenyl-3-[(phenylamino)carbonyl]-1H-pyrrole-1-heptanoic acid;
(2R-trans)-5-(4-Fluorophenyl)-2-(1-methylethyl)-N,4-diphenyl-1-[2-(tetrahydro-4-hydroxy-6-oxo-2H-pyran-2-yl)ethyl]-1H-pyrrole-3-carboxamide; and
3-[(4-Fluorophenyl)carbonyl]-2-(2-methyl-1-oxopropyl)-N,3-diphenyl-2-oxiranecarboxamide;
after storage at 25°C/60% relative humidity for 24 months.

8. A pharmaceutical composition according to claim 1 comprising amlodipine or pharmaceutically acceptable salts thereof and atorvastatin or pharmaceutically acceptable salts thereof and a carrier containing not more than 1% of a compound selected from the group consisting of:
2-(2-Amino-ethoxymethyl)-4-(2-chloro-phenyl)-6-methyl-pyridine-3,5-dicarboxylic acid 3-ethyl ester 5-methyl ester; and
6-(2-Chloro-phenyl)-8-methyl-3,4,6,7-tetrahydro-2H-1,4-benzoxazine-5,7-dicarboxylic acid 5-ethyl ester 7-methyl ester;
after storage at 25°C/60% relative humidity for 24 months.

9. A method for preparing a pharmaceutical composition according to claim 1 comprising:
[A] An atorvastatin granulation comprising:
Step (1) - dissolving a surface active agent in water and adding and hydrating a binder;
Step (2) - mixing atorvastatin calcium, an alkalising agent that forms a pH greater than 5, a filler/diluent, a filler/diluent/disintegrant agent, and a disintegrating agent in a granulating apparatus;
Step (3) - granulating the powder mix from Step (2) with the solution from Step (1) in the granulating apparatus; and
Step (4) - drying the granulation in a drying apparatus.
[B] A final formulation comprising:
Step (1) - adding amlodipine besylate, a filler/diluent, a disintegrating agent, and a glidant to the atorvastatin granulation;
Step (2) - passing the powder mixture through a mill; and
Step (3) - blending the milled powder mixture and a lubricating agent in a blender to afford a uniformly blended pharmaceutical composition for a solid dosage form.

10. A pharmaceutical composition according to claim 1 for use as a medicament.

11. The use of a pharmaceutical composition according to claim 1 for the manufacture of a medicament for the treatment of angina pectoris, atherosclerosis or combined hypertension and hyperlipidemia, or for the management of cardiac risk.
